# EUROPEAN PATENT APPLICATION

(11) **EP 0 806 422 A2**
(43) Date of publication of application: **12.11.1997**
(21) Application number: 97303059.6
(22) Date of filing: 06.05.1997
(51) Int. Cl.: C07D 403/04, C07D 239/42, C07D 239/52, A61K 31/505

(54) **Anti-cataract agent**

(30) Priority: 10.05.1996 JP 140659/96
(71) Applicant: Senju Pharmaceutical Co., Ltd., Osaka-shi, Osaka 541 (JP)
(72) Inventor: Inoue, Jun, 603, Rui-Shatore Suma Myodani, Kobe-shi, Hyogo 654-01 (JP)
(74) Representative: Burford, Anthony Frederick

(57) **Abstract**

Compounds represented by the general Formula (I); 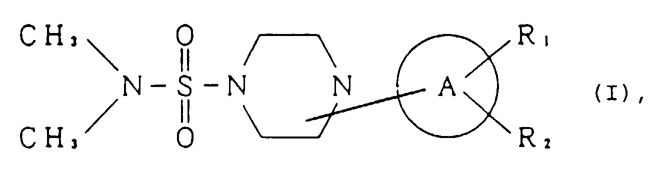 wherein A represents a pyrimidine ring, R₁ and R₂ represent independently hydrogen or optionally substituted C₁₋₆ alkyl or alkoxy and salts thereof are effective for preventing or treating cataract by oral or parenteral administration. The pyrimidine ring can be attached to the piperazine ring at a carbon or nitrogen atom of the piperazine ring.

## Description

The present invention relates to an anti-cataract agent which contains a piperazine derivative or its salt as an effective ingredient, and to novel piperazine derivatives and salts.

Cataract is one of the diseases occurring in highest frequency among lens disease. Cloudy state of lens is called cataract regardless of cause and in practice it is called by many classified names. Depending on causes, they are divided into congenital cataract and postnatal cataract contributed by some causes. Postnatal cataract is further divided, depending on symptoms or causes, as senile cataract in which lens becomes cloudy by aging without clear cause; traumatic cataract occurring by contusion, perforation, or flying in of a foreign body; cataract caused by abnormal metabolism such as diabetic cataract; or cataract caused by a medicine or poison such as steroid cataract. Cataract is also occasionally classified as premature cataract, mature cataract, postmature cataract and Morgagnian cataract, depending on the degree of turbidity in the lens; or as wedge-, dot- or plate-shaped cataract depending on the shape of lens turbidity.

Potassium iodide, pirenoxine, sodium 5,12-dihydroazapentacene sulfonate, glutathion, tiopronin, parotin (salivary gland hormone), cinerlia and other commercially available materials have been used to treat cataract. However, use of these materials does not cause disappearance of lens turbidity, but suppresses progress of lens turbidity. Furthermore, the reaction mechanisms or clinical effects of some of these medicines are difficult to prove scientifically and accurately.

Many researchers have studied the cause of cataract and developed medicines to prevent and treat cataract. Particularly, various medicines have been developed from a view point that aldose-reductase inhibitor may be effective for treating diabetic cataract. However, cataract is not a problem that can be solved by merely aldose-reductase inhibitor.

The present Inventors have found that piperazine derivatives represented by the following general Formula (I) have strong activity to delay the progress of cataract, but exhibit little or no inhibition activity to aldose-reductase.

The present invention relates to the use, as an anti-cataract agent, of a compound represented by the general Formula (I); 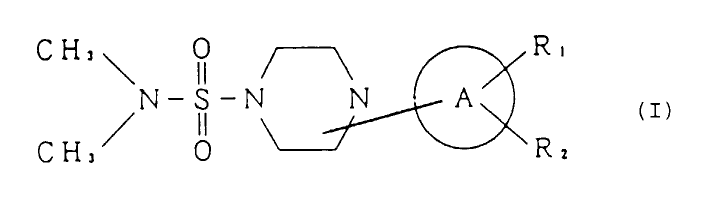 wherein A represents a pyrimidine ring, R₁ and R₂ represent independently a hydrogen atom, or C₁₋₆ alkyl or alkoxy group which may be substituted), or a salt thereof. Some of the compounds of Formula (I) are novel.

The lower alkyl group or moiety represented by R₁ and R₂ in the above formula can be straight- or branched-chain, such as methyl, ethyl, propyl, iso-propyl, sec-butyl, tert-butyl, pentyl, iso-pentyl, neo-pentyl, tert-pentyl, and hexyl, and may be substituted by, for example, halogen or hydroxy, such as trifluoromethyl and hydroxymethyl.

Preferred lower alkoxy groups, represented by R₁ and R₂ in the above formula, are C₁₋₄ alkoxy, such as methoxy, ethoxy, propoxy, and butoxy.

The salts of the compound represented by Formula (I) include physiologically acceptable salts with an inorganic or organic base, an inorganic or organic acid, or a basic or acidic aminoacid. As preferred examples of salts with an inorganic base, there may be exemplified salts with alkali metal such as sodium or potassium salts; salts with an alkali earth metal such as calcium or magnesium salts; aluminium or ammonium salts. Preferred examples of salts with an organic base include those with trimethylamine, pyridine, picoline, ethanolamine, diethanolamine, triethanolamine, dicyclohexylamine, or N,N-dibenzylethylenediamine. Preferred examples of salts with an inorganic acid include those with hydrochloric acid, hydrobromic acid, nitric acid, sulfuric acid or phosphoric acid.

As preferred salts with an organic acid, there can be exemplified salts with formic acid, trifluoroacetic acid, fumaric acid, oxalic acid, tartaric acid, maleic acid, citric acid, succinic acid, malic acid, methanesulfonic acid, benzenesulfonic acid, or p-toluenesulfonic acid. As preferred salts with a basic aminoacid, there can be exemplified salts with aspartic acid or glutamic acid.

The compounds and salts of Formula (I) are useful for preventing and treating cataract caused by various causes, for example diabetes or aging. They are suitably administered orally or parenterally for treating cataract and preventing the progress of cataract. Preparation forms for such administration are, for example, solid preparations such as tablets, pills, powder, capsules, or ointment, or liquid preparation such as injections, syrup, or ophthalmic solution. Any of these preparations may be prepared by known procedures. To those preparations, there may be suitably added various additives usually employed, such as excipient, binder, disintegrator, thickener, dispersant, absorption-promoting agent, buffer, surfactant, preservative, isotonicity-making agent, stabilizer, and pH-adjuster.

The compounds and salts of Formula (I) usually are administered to an adult by injection at 0.1-100 mg once a day, preferably 1-50 mg once a day; orally at 50-2000 mg three times a day, preferably 100-2000 mg three times a day; or as an ophthalmic solution at 2-6 drops per time several times a day in a concentration of usually 0.001-1.0 w/v %, preferably 0.01-0.5 w/v %. However, the dose varies depending on the age, body weight and symptoms of the subject, as well as the route of administration.

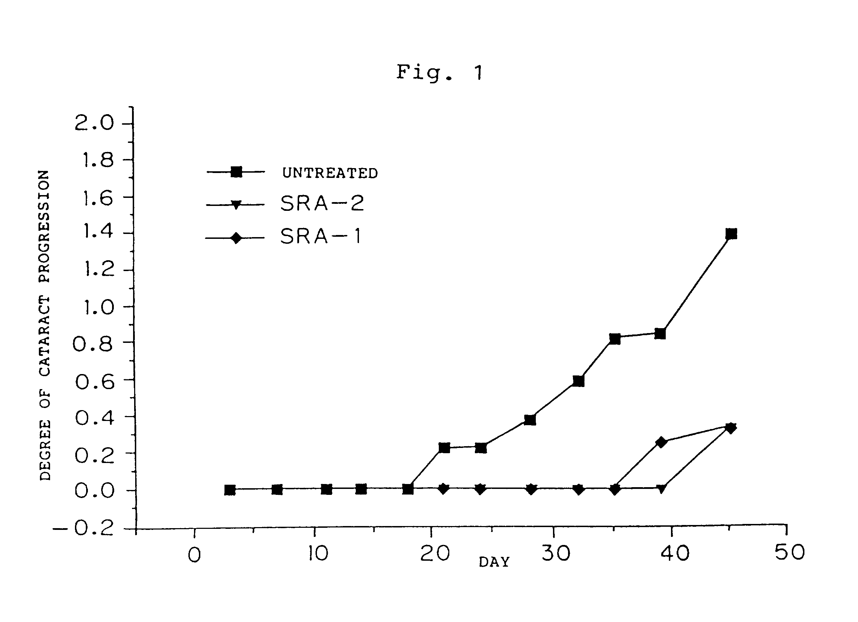

Figure 1 is a graph showing the result of Test Example 2. In the figure, SRA-1 is the product of Example 1 and SRA-2 is that of Example 3. As apparent from the figure, cataract formation is inhibited by administering these products.

The present invention is described in further detail by the following Examples, Test Examples and Preparation Examples, but is not limited thereby.

### Example 1

Triethylamine (15.3 ml, 0.11 mol) was added to a solution of 4-chloro-2,6-dimethoxypyrimidine (17.46 g, 0.1 mol) and l-dimethylsulfamoylpiperazine (21.26 g, 0.11 mol) in tetrahydrofuran, and the mixture was refluxed for 40 hours. After standing to cooling, deposited crystals were removed by filtration and the filtrate was concentrated and then purified by silica gel column-chromatography to give 4-((4-N,N-dimethylsulfamoyl)piperazino)-2,6-dimethoxypyrimidine (21.7 g, 65.6%) .
1HNMR (CDCl₃) 2.86 (s, 6H, -N-CH₃), 3.30 (m, 4H, piperazine ring), 3.91 (s, 3H, -O-CH₃), 3.92 (s, 3H, -O-CH₃), 5.52 (s, aromatic).

| | | | |
|---|---|---|---|
| Anal. Calculated (C₁₂H₂₁N₅O₄S) | C, 43.49; | H, 6.39; | N, 21.13. |
| Found | C, 43.38; | H, 6.25; | N, 21.04. |

### Example 2

To a solution of 2-chloro-4-trifluoromethylpyrimidine (10 g, 0.55 mol) and dimethylsulfamoylpiperazine (11.21 g, 0.058 mol) tetrahydrofuran (70 ml), was added triethylamine (7.82 ml, 0.056 mol) and the solution was refluxed for 8 hours. After cooling by standing, the reaction mixture was poured into cool water and extracted by ethyl acetate. The extract solution was washed with water and then saturated saline, and dried on magnesium sulfate, followed by solvent removal by distillation under reduced pressure. The residue was recrystallized in ethyl acetate:hexane (1:1) to give 2-((4-N,N-dimethylsulfamoyl)piperazino)-4-trifluoromethylpyrimidine (12.2 g, 65.4 %) .
1HNMR (CDCl₃) 2.86 (s, 6H, -N-CH₃), 3.32 (m, 4H, piperazine ring), 4.00 (m, 4H, piperazine ring), 6.82 (s, 1H, J=4.6 Hz, aromatic), 8.52 (s, 1H, J=4.6 Hz, aromatic).

| | | | |
|---|---|---|---|
| Anal. Calculated (C₁₂H₂₁N₅O₄S) | C, 38.93; | H, 4.75; | N, 20.64. |
| Found | C, 39,16; | H, 4.68; | N, 20.70. |

### Example 3.

Dimethylsulfamoylchloride (16.1 ml, 0.15 mol) was added to a suspension of 1-(2-pyrimidinyl)piperazine.2HCl (2347 g, 0.1 mol) in pyridine (200 ml), and the mixture was stirred for 6 hours at a temperature of 50 to 60°C. After cooling, the reaction mixture was poured into cool water. Deposited crystals were collected by filtration,
recrystallized in ethyl acetate:hexane (1:1) to give (1-N,N-dimethylsulfamoyl-4-(2-pyrimidinyl)piperazine 15.6 g, 57.6%).
1HNMR (CDCl₃) 2.36 (s, 6H, -N-CH₃) 3.30 (m, 4H, piperazine ring), 3.91 (m, piperazine ring), 6.51 (t, 1H, J=4.9 Hz, aromatic), 8.32 (d, 2H, J=4.9 Hz, aromatic) .

| | | | |
|---|---|---|---|
| Anal. Calculated (C₁₀H₁₇N₅O₂S) | C, 44.26; | H, 6.31; | N, 25.81. |
| Found | C, 44.41; | H, 6.20; | N, 25.78. |

### Preparation Example 1. (Tablets)

| | |
|---|---|
| Product compound of Example 1 | 30 mg |
| Lactose | 80 mg |
| Starch | 17 mg |
| Magnesium stearate | 3 mg |

Employing the above ingredients as the material per tablet, tablets are formed by conventional method. Sugar may be coated on the tablets, if necessary.

### Preparation Example 2. (Injections)

| | |
|---|---|
| Product compound of Example 2 | 100 mg |
| Sodium chloride | 900 mg |
| 1-N sodium hydroxide | suitable amount (pH 6.8) |

The above ingredients are mixed by conventional method to make a solution for injection. The solution is poured dividedly into 2 ml glass ampoules and sealed.

### Preparation Example 3. (Ophthalmic solution)

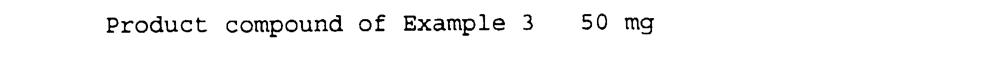

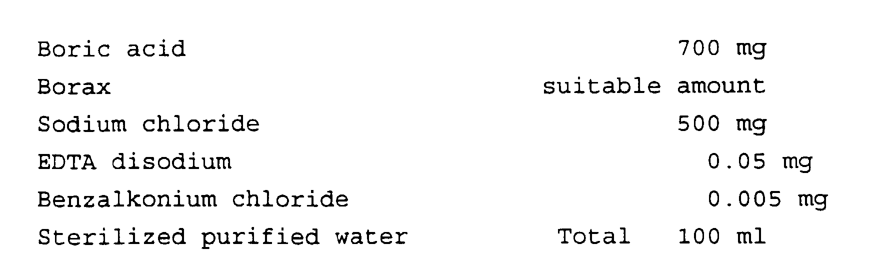

The above ingredients are mixed by conventional method to make a ophthalmic solution.

### Test Example 1.

### Enzyme Assay

The activity of purified recombinant rat aldose reductase (Old SE, Sato S, Kador PF. and Carper DA.: In vitro expression of rat lens aldose reductase in Escherichia coli., Proc. Nat. Acad. Sci. USA, 87:4942-4945, 1990) was assayed by spectrophotometrically monitoring decrease in absorbance of NADPH (reduced nicotinamide adenine dinucleotide phosphate) at 340 nm (Kador PF., Goosey JD., Sharpless NE., Kolish J, and Miller DD.: Stereoscopic inhibition of aldose reductase, European J., Med. Chem. 16: 293-298, 1981).

One enzyme unit was defined as amount of enzyme consuming one micromole of NADPH under the assay conditions.

### Result

With reference to 1-N,N-dimethylsulfamoyl-4-(2-pyrimidinyl)piperazine (SRA-1; product of Example 3) and 4-((4-dimethylsulfamoyl)piperazino)-2,6-dimethoxypyrimidine (SRA-2; product of Example 1), their in vitro ability to inhibit rat lens aldose reductase were determined. Their inhibition rate of aldose reductase were very low as 5% with SRA-1 and 13% with SRA-2 in the same 1x10⁻⁴ M concentration.

### Test Example 2.

### Diabetic Rats

Diabetes was induced in 40 young (60-80 g) Sprague Dawley rats by tail vein injection of 75 mg/kg streptozotocin in Tris-HCl and divided into 3 groups. After 4 days all rats were tested with glucostrips (tail blood), and those with blood sugar levels less than 250 mg % were reinjected with 70 mg/kg streptozotocin dissolved in Tris-HCl.

Drug administration was carried out by adding 0.06% drug to rat chow. The rat chow with added drug was formed by mixing appropriate amounts of each drug with powdered rat chow and then cold pressing the mixture into different size pellets for identification. The rat chow was commercially prepared by BioServe (Frenchtown, N.J.). Subsequently, each compound was dissolved in 500 ml of Tris buffer and mixed with 5 kg of standard rat chow pellets until all liquid was absorbed into the pellets and the wet pellets were dried for 24 hours at 60°C. Rat chow not containing drug was fed to a control group of rats.

### Cataract Formation

Lens changes were evaluated by portable slit lamp at approximately 3-6 day intervals in the diabetic rats. Lens changes were evaluated on a scale at 0 to 3 as follows: 0 clear lens; 1 vacuoles present; 2 cortical opacities; 3 hypermature cataract. The severity of lens changes within the vacuole and cortical stage was further subjectively defined as follows: 1.2 peripheral equatorial vacuoles; 1.4 obvious formation 50% of the lens; 1.6 obvious vacuole formation 50% of lens; 1.8 total lens covered by vacuoles; 2.2 start of cortical changes with posterior capsule still visible by slit lamp; 2.4 advanced cortical opacities covering most of lens; cortical opacity expanded to whole lens; 2.8 total lens is white in appearance.

### Result

In the diabetic lens changes were observed to begin 21 days after the injection of streptozotocin (Figure 1). Vacuoles formation was clearly visible by 35 days.

Treatment with SRA-1 and SRA-2 delayed the onset and severity of sugar cataract formation. Lens changes were observed at 39 days in the SRA-1 treated group and 45 days in the SRA-2 treated group and these did not progress to the vacuole stage. After 45 days, analysis of tail vein blood indicated that all rats had blood glucose values in excess of 250 mg%.

Thus, the present piperazine derivatives of Formula (I) or their salts can be utilized effectively for treating and/or preventing cataract of various causes, particularly human or animal cataract causing diabetes.

## Claims

1. A compound represented by the general Formula (I): 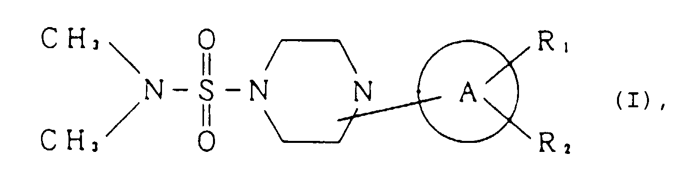 wherein A represents a pyrimidine ring, R₁ and R₂ represent independently a hydrogen atom, or C₁₋₆ alkyl or alkoxy group which may be substituted or a salt thereof.

2. A compound as claimed in Claim 1, wherein the pyrimidine ring is attached to the piperidine ring at the 4-position thereof (i.e. nitrogen atom).

3. A compound as claimed in Claim 1 or Claim 2, wherein R₁ and R₂ independently represent hydrogen, trifluoromethyl, C₁-C₄ alkoxy or C₁₋₆ alkyl group optionally substituted by halogen or hydroxyl

4. A compound as claimed in Claim 3, wherein R₁ and R₂ independently represent hydrogen, trifluoromethyl, methoxyl or hydroxymethyl.

5. 4-((4-N,N-dimethylsulfamoyl)piperazino)-2,6-dimethoxy pyrimidine or a salt thereof.

6. 2-((4-N,N-dimethylsulfamoyl)piperazino)-4-trifluoromethylpyrimidine or a salt thereof.

7. (1-N,N-dimethylsulfamoyl-4-(2-pyrimidinyl)piperazine or a salt thereof.

8. An anti-cataract composition comprising, as an active anti-cataract ingredient, a compound represented by the general Formula (I) as defined in Claim 1 or a physiologically acceptable salt thereof.

9. A composition as claimed in Claim 8, wherein said compound or salt is as defined in any one of Claims 2 to 7.

10. A composition as claimed in Claim 8 or Claim 9 in the form of an ophthalmic solution.

11. A compound represented by the general Formula (I) as defined in Claim 1 or a physiologically acceptable salt thereof for use in the treatment of the human or animal body by therapy.

12. A compound represented by the general Formula (I) as defined in Claim 1 or a physiologically acceptable salt thereof for use in the treatment or prevention of cataract.

13. A compound as claimed in Claim 11 or claim 12, wherein said compound or salt is as defined in any one of Claims 2 to 7.

15. The use of a compound represented by the general Formula (I) as defined in Claim 1 or a physiologically acceptable salt thereof in the manufacture of a medicament for the treatment or prevention of cataract.

16. A use as claimed in Claim 15, wherein said compound or salt is as defined in any one of Claims 2 to 7.
